(19) Europäisches Patentamt
European Patent Office
Office européen des brevets

(11) Publication number: **0 070 525**
**B1**

(12) **EUROPEAN PATENT SPECIFICATION**

(45) Date of publication of patent specification: **14.01.87**

(51) Int. Cl.$^4$: **A 61 K 9/08**, A 61 K 31/415

(21) Application number: **82106376.5**

(22) Date of filing: **15.07.82**

(54) **Fungicidal preparations for external use.**

(30) Priority: **22.07.81 JP 115817/81**

(43) Date of publication of application:
**26.01.83 Bulletin 83/04**

(45) Publication of the grant of the patent:
**14.01.87 Bulletin 87/03**

(84) Designated Contracting States:
**BE CH DE FR GB IT LI NL SE**

(56) References cited:
**EP-A-0 007 595**
**EP-A-0 011 769**
**EP-A-0 019 730**
**EP-A-0 022 969**
**EP-A-0 023 614**
**EP-A-0 031 883**
**DE-A-3 030 323**
**FR-A-2 015 913**
**FR-M-1 443**
**LU-A-83 073**
**US-A-4 123 542**
**CHEMICAL ABSTRACTS, vol. 84, no. 13, 29th March 1976, page 101, no. 84713e, Columbus, Ohio, USA, Y. SUENAGA: "New anti-fungal agents"**

**The file contains technical information submitted after the application was filed and not included in this specification**

(73) Proprietor: **Toko Yakuhin Industry Co., Ltd.**
**12-23, 2-chome, Honjyo-Nishi**
**Oyodo-ku Osaka (JP)**

(72) Inventor: **Kamishita, Takuzo**
**12-28, 6-chome, Tonda-cho**
**Takatsuki-shi Osaka (JP)**

(74) Representative: **Kraus, Walter, Dr. et al**
**Patentanwälte Kraus, Weisert & Partner**
**Thomas-Wimmer-Ring 15**
**D-8000 München 22 (DE)**

(56) References cited:
**Remington's Pharmaceutical Sciences (1975), 15th Ed., p. 1180**

**Chemical Abstracts 88, 158482f, Derwent JP Abstracts 11048A/06**

**EP 0 070 525 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to pharmaceutical preparations for external use. More specifically, this invention relates to a preparation for external use which is prepared by forming into a pharmaceutical preparation, with the use of a base for external use, a solution of an antifungal agent such as miconazole, econazole or isoconazole dissolved in a substance which can dissolve said antifungal agent and which can exert, when applied externally, a pharmaceutical activity as an adjuvant. This substance is selected from crotamiton and peppermint oil.

Previously, the inventor of this invention found that clotrimazole "1-[(2-chlorophenyl)diphenylmethyl]-1H-imidazole", a kind of antifungal, dissolves in crotamiton "crotonyl-N-ethyl-o-toluidine" and, based on this finding, prepared a gel preparation and so forth (US—A—4,267,169). From FR—M—1 443 it was known that crotamiton was a solvent for Hydrocortisone® and this was used in antimycotic preparations. Chemical Abstracts 88: 158 482 k (1978) describes crotamiton as a desirable solvent for the fungicide tolnaphthate. The use of benzyl alcohol as a possible solvent for certain imidazoles bearing a substituted phenyl group is described in EP—A—31 883, EP—A—22 969, EP—A—23 614, EP—A—11 769 and EP—A—19 730.

As a result of further investigation, the inventor of this invention has found that antifungal compounds, such as miconazole, econazole or isoconazole, which have an imidazole ring in their molecule in common with clotrimazole but are markedly different from the latter in the other parts of their molecular structure, will dissolve in crotamiton and peppermint oil, each of which have themselves pharmaceutical activities as a skin preparation for external use and further that an excellent preparation for external use can be obtained by forming a solution thus prepared into a pharmaceutical preparation.

This invention provides a pharmaceutical preparation for external use which comprises a soluton of a compound represented by the general formula (I):

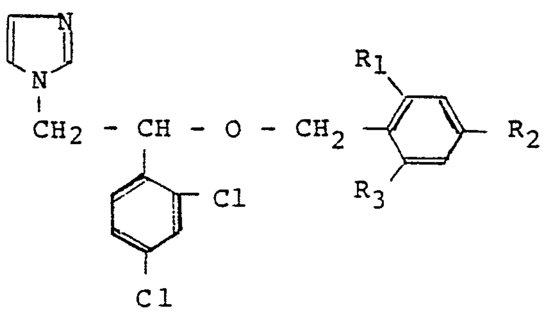

(I)

wherein at least one of $R_1$, $R_2$ and $R_3$ is chlorine atom and the rest thereof is hydrogen atom, which is dissolved in at least one adjuvant selected from peppermint oil and crotamiton in an amount at least sufficient for dissolving the compound (I), and a base for the preparation for external use.

The antifungal ingredient involved in this invention, is a compound represented by the general formula (I):

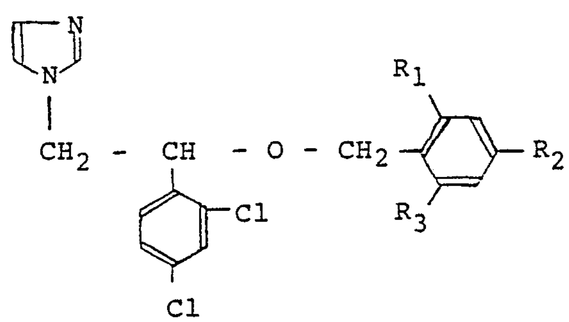

(I)

wherein at least one of $R_1$, $R_2$ and $R_3$ is chlorine atom and the rest thereof is hydrogen atom; it is miconazole when $R_1$ and $R_2$ are each chlorine atom and $R_3$ is hydrogen atom, isoconazole when $R_1$ and $R_3$ are each chlorine atom and $R_2$ is hydrogen atom, and econazole when $R_2$ is chlorine atom and $R_1$ and $R_3$ are each hydrogen atom.

All the stereoisomers of the compound (I) which has an asymmetric carbon in its molecule, can be used as the material for this invention. Preferable materials are econazole, miconazole and isoconazole.

The amount of the compound (I) contained in a preparation for external use of the invention in general is preferably 0.5—2%, however, it should be understood that amounts of, for example, 0.3%, 2.5% and 3% each are also included in the scope of the invention.

Peppermint oil used in the invention as an adjuvant contains 1-menthol as a main component. Although peppermint oil shows some difference in its components depending on the kind of the plants

from which it is obtained, all kinds thereof can be used. Thus there may be used all kinds of peppermint oil listed and defined in pharmacopeias of Japan, the United Kingdom, West Germany and the like.

When the adjuvant, peppermint oil or crotamiton, is used solely for dissolving the compound (I), it is enough to use the amount thereof sufficient to dissolve the compound (I). The minimum necessary amount thereof has proved to be generally about 1—2 parts by weight for every 1 part by weight of the compound (I). This invention includes cases where more than one of the adjuvants are used to utilize each supplementary pharmaceutical activity as an agent for external use. In such cases, the compound (I) may be dissolved in one of the adjuvants followed by addition of a desired amount of the other adjuvant.

Thus, this invention provides, as a preferred pharmaceutical preparation for external use, a preparation which comprises 0.5—2% of the compound (I) and 1—8% of one, or both kinds of adjuvants selected form peppermint oil and crotamiton.

The percentage used above and hereinafter are on a weight/weight basis, except where any specific explanation is given.

A combination of these 2 adjuvants may also be used in this invention.

According to one aspect of the invention, it provides a method for preparing an antifungal preparation for external use which comprises dissolving the compound (I) in at least one adjuvant mentioned above in an amount at least sufficient for dissolving said compound and then forming the resultant solution into a pharmaceutical preparation by using a base for external use.

To prepare the pharmaceutical preparation of the invention, it is necessary to dissolve beforehand the compound of the formula (I) in a sufficient amount of an adjuvant or adjuvants such as peppermint oil and so on for dissolution followed by forming the resulting solution into a pharmaceutical preparation by using a base for external use. When the compound (I) is directly formed into a pharmaceutical preparation with the use of a base for external use or when the compound is formed into a pharmaceutical preparation with the use of a mixture of a base for external use and said adjuvant(s), separation of crystals of the compound (I) is observed. In this invention, there is expected a supplementary drug action of peppermint oil and so forth themselves as an agent for external use, thus the drug action of the main ingredient, the antifungal ingredient, being enhanced.

Preferred dosage forms of the pharmaceutical preparation of the invention are a gel, a gel cream, a cream, and a liquid preparation. Bases for external use that are employed for the preparation of dosage forms may be the conventional ones which are known in the art. For example, as a base for gel preparations there may be mentioned a dilute aqueous solution of carboxyvinyl polymer and an aqueous solution of water soluble base (e.g., sodium hydroxide). With the use of such bases, gel preparations may be readily obtained by forming a solution of the compound (I) in peppermint oil and so forth into a pharmaceutical preparation.

As a base for cream preparations there may be mentioned the abovementioned base for gel preparation plus an emulsifying agent such as a nonionic surface active agent and an oily substance such as liquid paraffin.

The carboxyvinyl polymer employed here is a hydrophilic polymer which is obtained by polymerizing acrylic acid as a main component and is commercially available from, for example, Goodrich Chemical Corp. under the trade name of Carbopol 934, 940 and 941 or from Wako Pure Chemical Industries, Ltd. of Japan under the trade name of Hiviswako 103, 104, 105, etc.

Preferable example of the cream base is Hydrophilic Ointment which is listed in Japanese Pharmacopeia.

Preferable example of a base for liquid preparations is a mixture of ethanol and water.

Besides, substances cited above as examples, other bases for external use, preservatives, and other additives that are known in the art may be suitably selected for use. Conditions for the preparation of the pharmaceutical preparation for external use may also be selected as well.

Thus, when peppermint oil is used in the pharmaceutical preparation of the invention, the preparation has an antipruritic acitivity resulting from hypesthesia by peppermint oil, and the penetration of the effective ingredient into keratin is made easier owing to the dissolution of the active ingredient in peppermint oil, peppermint oil thus giving a refreshing feeling to the skin. Crotamiton also shows antipruritic acitivity.

The invention will next be illustrated by the following examples, although it is not limited thereby.

## Example 1

Liquid Preparations

(1) Miconazole containing liquid preparations for external use

The liquid preparations of which components are shown in Table 1 were prepared in the manner mentioned below.

Miconazole was dissolved in an adjuvant or a mixture of adjuvants prepared beforehand, with heating to about 70—80°C. Aqueous 95% ethanol was added by portions with stirring and then purified water was slowly added to obtain the liquid preparation.

TABLE 1

| Component | (weight/weight %) | | | | | | | | |
|---|---|---|---|---|---|---|---|---|---|
| Miconazole | 0.5 | 0.5 | 1.0 | 1.0 | 1.0 | 1.0 | 2.0 | 2.0 | 2.0 |
| Adjuvant | | | | | | | | | |
| Peppermint oil | 5.0 | | 1.0 | 5.0 | | | 6.0 | | |
| Crotamiton | | 5.0 | | | 5.0 | | | 6.0 | 6.0 |
| Base | | | | | | | | | |
| Aqueous 95% ethanol | 40.0 | 40.0 | 50.0 | 45.0 | 45.0 | 45.0 | 50.0 | 45.0 | 50.0 |
| Purified water | 54.5 | 54.5 | 48.0 | 49.0 | 49.0 | 49.0 | 42.0 | 47.0 | 42.0 |

(2) Econazole or Isoconazole containing liquid preparations for external use.

The liquid preparations having the components which are shown in the Tables 2 and 3 where prepared in the same manner as in Example 1, (1) except to use econazole or isoconazole instead of miconazole.

TABLE 2

| Component | (weight/weight %) | |
|---|---|---|
| Econazole | 0.5 | 2.0 |
| Adjuvant | | |
| Peppermint oil | 1.0 | |
| Crotamiton | | 5.0 |
| Base | | |
| Aqueous 95% ethanol | 40.0 | 55.0 |
| Purified water | 58.5 | 38.0 |

TABLE 3

| Component | (weight/weight %) | |
|---|---|---|
| Isoconazole | 0.5 | 2.0 |
| Adjuvant | | |
| Peppermint oil | 1.0 | |
| Crotamiton | | 5.0 |
| Base | | |
| Aqueous 95% ethanol | 40.0 | 55.0 |
| Purified water | 58.5 | 38.0 |

Example 2

Gel preparations

(1) Miconazole containing gel preparations for external use

The gel preparations having the components which are shown in the Table 4 were prepared in the same manner mentioned below.

Miconazole was dissolved in an adjuvant or a mixture of adjuvants prepared beforehand, with heating to about 70—80°C.

Aqueous 20% sodium hydroxide solution was slowly added into aqueous 4% Carbopol solution with sufficient stirring and purified water was added and further sufficiently stirred. The miconazole solution was added into the above resulting liquid followed by enough stirring to homogenize the whole liquid to obtain the gel preparation.

TABLE 4

| Component | (weight/weight %) | | | | | |
|---|---|---|---|---|---|---|
| Miconazole | 0.5 | 0.5 | a* 1.0 | c* 1.0 | 2.0 | 2.0 |
| Adjuvant | | | | | | |
| Peppermint oil | 1.0 | | 2.0 | | 4.0 | |
| Crotamiton | | 1.0 | | 2.0 | | 4.0 |
| Base | | | | | | |
| Aqueous 4% Carbopol solution | 30.0 | 30.0 | 38.6 | 38.6 | 30.0 | 30.0 |
| Aqueous 20% sodium hydroxide solution | 2.4 | 2.4 | 2.6 | 2.6 | 2.4 | 2.4 |
| Purified water | 66.1 | 66.1 | 55.8 | 55.8 | 61.6 | 61.6 |

* Each the preparations a and c shows a pH of 6.36 at 25°C and a viscosity of 58000 cp.

(2) Econazole or isoconazole containing gel preparation for external use.

The gel preparations having the components which are shown in the below Tables 5 and 6 were prepared in the same manner as in Example 2, (1) except to use econazole or isoconazole instead of miconazole.

TABLE 5

| Component | (weight/weight %) | |
|---|---|---|
| Econazole | 1.0 | 1.0 |
| Adjuvant | | |
| Peppermint oil | 2.0 | |
| Crotamiton | | 5.0 |
| Base | | |
| Aqueous 4% Carbopol solution | 30.0 | 30.0 |
| Aqueous 2% sodium hydroxide solution | 25.0 | 25.0 |
| Purified water | 42.0 | 39.0 |

TABLE 6

| Component | (weight/weight %) | |
|---|---|---|
| Isoconazole | 1.0 | 1.0 |
| Adjuvant | | |
| Peppermint oil | 2.0 | |
| Crotamiton | | 5.0 |
| Base | | |
| Aqueous 4% Carbopol solution | 30.0 | 30.0 |
| Aqueous 2% sodium hydroxide solution | 25.0 | 25.0 |
| Purified water | 42.0 | 39.0 |

Example 3

Cream preparations

(1) Miconazole containing cream preparations for external use

The cream preparations having the components which are shown in the below Table 7 were prepared in the manner mentioned below.

Miconazole was dissolved in an adjuvant or a mixture of adjuvants prepared beforehand, with heating to about 70—80°C. Stearic acid, cetanol, isopropyl myristinate, white vaseline, polyoxy ethylene sorbitan monostearate and sorbitan monostearate were added and heated to about 70—80°C in a water bath.

A mixture of sodium lauryl sulfate and purified water was heated to about 70—80°C in a water bath to obtain a solution. This solution was added into the above miconazole containing liquid followed by sufficient stirring and cooling to obtain the cream preparation.

TABLE 7

| Component | (weight/weight %) | | | | | |
|---|---|---|---|---|---|---|
| Miconazole | 0.5 | 0.5 | 1.0 | 1.0 | 2.0 | 2.0 |
| Adjuvant | | | | | | |
| Peppermint oil | 1.5 | | 3.0 | | 6.0 | |
| Crotamiton | | 1.5 | | 3.0 | | 5.0 |
| Base | | | | | | |
| Stearic acid | 5.0 | 5.0 | 5.0 | 5.0 | 5.0 | 5.0 |
| Cetanol | 5.0 | 5.0 | 5.0 | 5.0 | 5.0 | 5.0 |
| Isopropyl myristinate | 10.0 | 10.0 | 10.0 | 10.0 | 10.0 | 10.0 |
| White vaseline | 3.0 | 3.0 | 3.0 | 3.0 | 3.0 | 3.0 |
| Liquid paraffin | 5.0 | 5.0 | 5.0 | 5.0 | 5.0 | 5.0 |
| Polyoxyethylensorbitan monostearate | 3.3 | 3.3 | 3.3 | 3.3 | 3.3 | 3.3 |
| Sorbitan monostearate | 1.0 | 1.0 | 1.0 | 1.0 | 1.0 | 1.0 |
| Sodium lauryl sulfate | 0.2 | 0.2 | 0.2 | 0.2 | 0.2 | 0.2 |
| Purified water | 65.5 | 65.5 | 63.5 | 63.5 | 59.5 | 60.5 |

(2) Econazole or isoconazole containing cream preparations for external use.

The cream preparations having the components which are shown in the below Tables 8 and 9 were prepared in the same manner as in Example 3, (1) except to use econazole or isoconazole instead of miconazole.

TABLE 8

| Component | (weight/weight %) | |
|---|---|---|
| Econazole | 1.0 | 2.0 |
| Adjuvant | | |
| Peppermint oil | | 8.0 |
| Crotamiton | 3.0 | |
| Base | | |
| Stearic acid | 5.0 | 5.0 |
| Cetanol | 5.0 | 5.0 |
| Isopropyl myristinate | 10.0 | 10.0 |
| White vaseline | 3.0 | 3.0 |
| Liquid paraffin | 5.0 | 5.0 |
| Polyoxyethylenesorbitan monostearate | 3.3 | 3.3 |
| Sorbitan monostearate | 1.0 | 1.0 |
| Sodium lauryl sulfate | 0.2 | 0.2 |
| Purified water | 63.5 | 57.5 |

TABLE 9

| Component | (weight/weight %) | |
|---|---|---|
| Isoconazole | 1.0 | 2.0 |
| Adjuvant | | |
| Peppermint oil | | 8.0 |
| Crotamiton | 3.0 | |
| Base | | |
| Stearic acid | 5.0 | 5.0 |
| Cetanol | 5.0 | 5.0 |
| Isopropyl myristinate | 10.0 | 10.0 |
| White vaseline | 3.0 | 3.0 |
| Liquid paraffin | 5.0 | 5.0 |
| Polyoxyethylenesorbitan monostearate | 3.3 | 3.3 |
| Sorbitan monostearate | 1.0 | 1.0 |
| Sodium lauryl sulfate | 0.2 | 0.2 |
| Purified water | 63.5 | 57.5 |

Example 4

Gel cream preparations

(1) Miconazole containing gel cream preparations for external use

The gel cream preparations having the components which are shown in the below Table 10 were prepared in the manner mentioned below.

Miconazole was dissolved in an adjuvant or a mixture of adjuvants prepared beforehand, with heating to about 70—80°C. Isopropyl myristinate, liquid paraffin and lauromacrogol were added and the resultant was heated to about 70—80°C in a water bath.

Purified water was added into aqueous 4% Carbopol solution with stirring and aqueous 20% sodium hydroxide solution was added and then the resultant was heated to about 70—80°C in a water bath.

The above miconazole solution was added into the above Carbopol containing mixture followed by sufficient stirring and cooling to obtain the gel cream preparation.

TABLE 10

| Component | (weight/weight %) | | | | | |
|---|---|---|---|---|---|---|
| | | *b' | *c' | | | |
| Miconazole | 0.5 | 0.5 | 1.0 | 1.0 | 2.0 | 2.0 |
| Adjuvant | | | | | | |
| Peppermint oil | 1.0 | | 2.0 | | 6.0 | |
| Crotamiton | | 1.0 | | 2.0 | | 6.0 |
| Base | | | | | | |
| Isopropyl myristinate | 5.0 | 5.0 | 5.0 | 5.0 | 5.0 | 5.0 |
| Liquid paraffin | 5.0 | 5.0 | 5.0 | 5.0 | 5.0 | 5.0 |
| Lauromacrogol | 1.0 | 1.0 | 1.0 | 1.0 | 1.0 | 1.0 |
| Aqueous 4% Carbopol solution | 30.0 | 30.0 | 30.0 | 30.0 | 30.0 | 30.0 |
| Aqueous 2% sodium hydroxide solution | 2.0 | 2.0 | 2.0 | 2.0 | 2.0 | 2.0 |
| Purified water | 55.5 | 55.5 | 54.0 | 54.0 | 49.0 | 49.0 |

*b' pH (at 25°C) 6.30 Viscosity (cp) 46,000
*c' pH (at 25°C) 6.35 Viscosity (cp) 43,000

Stability test of the preparations

The stability of the miconazole (1%) gel preparation in Example 2 which is marked with (a) in Table 4 or the miconazole (1%) gel cream preparation in Example 4 which is marked with (c') in Table 10 was determined by analyzing the concentration of the antifungal agent in each of the preparation after standing for a maximum of 90 days at a temperature of 40, 50, or 60°C by using the high pressure liquid chromatograph (Column: LC—3A, spectorophotometer: SPD—2A, Simadzu Corporation in Japan). The results were shown in the Tables 11 and 12.

Further, no changes of the dosage form or decomposition of the antifungal agent in the abovementioned preparations were observed after standing for 90 days at room temperature.

TABLE 11

The miconazole (1%) gel preparation (a) in Example 2

| Period (day) | Temperature (°C) | Ratio of miconazole detected to miconazole added in the preparation (%) |
|---|---|---|
| 0 | | 99.3 |
| 30 | 40 | 100.0 |
| | 50 | 99.6 |
| | 60 | 100.5 |
| 60 | 40 | 99.8 |
| | 50 | 100.3 |
| | 60 | 100.1 |
| 90 | 40 | 99.9 |
| | 50 | 100.0 |
| | 60 | 99.6 |

TABLE 12

The miconazole (1%) gel cream preparation (c') in Example 4

| Period (day) | Temperature (°C) | Ratio of miconazole detected to miconazole added in the preparation (%) |
|---|---|---|
| 0 | | 100.5 |
| 30 | 40 | 100.0 |
| | 50 | 99.3 |
| | 60 | 99.8 |
| 60 | 40 | 99.7 |
| | 50 | 100.4 |
| | 60 | 100.1 |
| 90 | 40 | 100.4 |
| | 50 | 99.7 |
| | 60 | 100.2 |

**Claims**

1. A pharmaceutical preparation for external use which comprises a solution of a compound represented by the general formula (I):

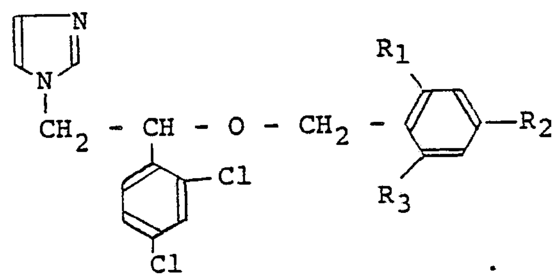

(I)

.

wherein at least one of $R_1$, $R_2$ and $R_3$ is a chlorine atom and the rest thereof is a hydrogen atom dissolved in at least one adjuvant selected from peppermint oil and crotamiton in an amount at least sufficient for dissolving the compound (I), and a base for the preparation for external use.

2. A pharmaceutical preparation according to claim 1 in which the compound (I) is miconozole, econazole or isoconazole.

3. A pharmaceutical preparation according to claim 1 or 2 which contains 0.5—2 w/w% of the compound (I).

4. A pharmaceutical preparation according to claim 1 which contains 1—8 w/w% of the adjuvant.

5. A pharmaceutical preparation according to claim 4 in which contains only one adjuvant.

6. A pharmaceutical preparation according to claim 1 in which the amount of the adjuvant at least sufficient to dissolve the compound (I) is from one to two parts by weight to one part by weight of the compound (I).

7. A pharmaceutical preparation according to claim 1 which is in a gel, gel cream, cream or liquid form.

**Patentansprüche**

1. Pharmazeutische Zubereitung für den äußerlichen Gebrauch, dadurch gekennzeichnet, daß sie eine Lösung einer Verbindung der allgemeinen Formel (I):

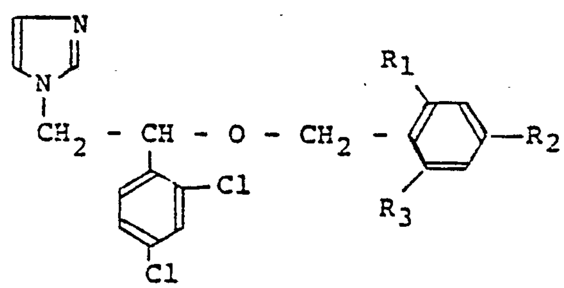

(I)

worin mindestens einer der Substituenten $R_1$, $R_2$ und $R_3$ ein Chloratom und der Rest ein Wasserstoffatom bedeuten, gelöst in mindestens einem Adjuvans, ausgewählt unter Pfefferminzöl und Chrotamiton, in einer Menge, die mindestens ausreicht, die Verbindung (I) zu lösen, und einen Grundstoff für die Zubereitung für die äußere Anwendung enthält.

2. Pharmazeutische Zubereitung nach Anspruch 1, dadurch gekennzeichnet, daß die Verbindung (I) Miconozol, Econozol oder Isoconazol ist.

3. Pharmazeutische Zubereitung nach Anspruch 1 oder 2, dadurch gekennzeichnet, daß sie 0,5 bis 2 Gew./Gew.-% der Verbindung (I) enthält.

4. Pharmazeutische Zubereitung nach Anspruch 1, dadurch gekennzeichnet, daß sie 1 bis 8 Gew./Gew.-% des Adjuvans enthält.

5. Pharmazeutische Zubereitung nach Anspruch 4, dadurch gekennzeichnet, daß sie nur ein Adjuvans enthält.

6. Pharmazeutische Zubereitung nach Anspruch 1, dadurch gekennzeichnet, daß die Menge an Adjuvans, die mindestens ausreicht, die Verbindung der Formel (I) zu lösen, 1 bis 2 Gew.-Teile, bezogen auf 1 Gew.-Teil der Verbindung (I), beträgt.

7. Pharmazeutische Zubereitung nach Anspruch 1, dadurch gekennzeichnet, daß sie in Form eines Gels, einer gelartigen Creme, einer Creme oder in flüssiger Form vorliegt.

**Revendications**

1. Préparation pharmaceutique à usage externe, caractàrisée en ce qu'elle comprend une solution d'un composé représenté par la formule générale I:

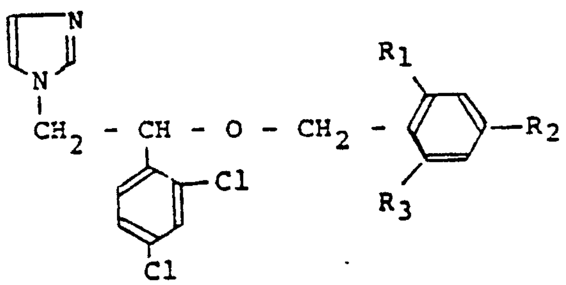

(I)

dans laquelle au moins un des symboles $R_1$, $R_2$ et $R_3$ est un atome de chlore et le(s) autre(s) un atome d'hydrogène, dissous dans au moins un adjuvant choisi parmi l'essence de menthe et le crotamiton en une quantité au moins suffisante pour dissoudre le composé (I), et une base pour la préparation à usage externe.

2. Préparation pharmaceutique selon la revendication 1, caractérisée en ce que le composé (I) est le miconazole, l'éconazole ou l'Isoconazole.

3. Préparation pharmaceutique selon la revendication 1 ou 2, caractérisée en ce qu'elle contient 0,5 à 2% (poids/poids) du composé (I).

4. Préparation pharmaceutique selon la revendication 1, caractérisée en ce qu'elle contient 1 à 8% (poids/poids) de l'adjuvant.

5. Préparation pharmaceutique selon la revendication 4, caractérisée en ce qu'elle ne contient qu'un seul adjuvant.

6. Préparation pharmaceutique selon la revendication 1, caractérisée en ce que la quantité de l'adjuvant au moins suffisante pour dissoudre le composé (I) est de une à deux parties par poids à une partie par poids du composé (I).

7. Préparation pharmaceutique selon la revendication 1, caractérisée en ce qu'elle se trouve sous forme d'un gel, d'une gel-crème, d'un crème ou d'un liquide.